# EUROPEAN PATENT APPLICATION

(11) **EP 2 338 331 A1**
(43) Date of publication of application: **29.06.2011**
(21) Application number: 09180492.2
(22) Date of filing: 22.12.2009
(51) Int. Cl.: A01N 25/34, A01N 63/00, A01N 63/02, A01N 63/04, A01N 65/00, D06M 16/00

(54) **Methods and compositions for biologically controlling microbial growth on clean room equipment**

(71) Applicant: Research Center Pharmaceutical Engineering GmbH, 8010 Graz (AT)
(72) Inventor: Berg, Gabriele, 8010 Graz (AT); Hartenberger, Kathrin, 8020 Graz (AT); Liebminger, Stefan, 8020 Graz (AT)
(74) Representative: Dilg, Haeusler, Schindelmann Patentanwaltsgesellschaft mbH

(57) **Abstract**

The present invention relates to methods and compositions for controlling microbial growth on clean room equipment, preferably on clean room clothing. More particularly, the invention is directed to a corresponding method comprising contacting the clean room equipment to be treated with one or more antimicrobial agents; and incubating the clean room equipment for a pre-selected period of time, wherein the one or more antimicrobial agents are of organic origin. In a further aspect, the invention relates to a kit-of-parts, comprising one or more antimicrobial agents selected from the group consisting of microorganisms, preferably bacteria and fungi, and metabolites produced by said microorganisms for performing a method as defined herein.

## Description

### FIELD OF THE INVENTION

The present invention relates to methods and compositions for controlling microbial growth on clean room equipment, preferably on clean room clothing. More particularly, the invention is directed to means for the microbial decontamination of such clean room equipment by employing one or more antimicrobial agents of organic origin.

### BACKGROUND

Clean room environments are of immense value in the fabrication and assembly of many industrial products, including *inter alia* electronics, semiconductors, spacecraft components, medical devices, pharmaceuticals, and cosmetics. The low density of environmental pollutants such as dust, aerosolized particulates, airborne microorganisms, and chemical vapors within these clean room facilities reduces the amount of both inorganic and organic (i.e. biological) contaminations on and within the products manufactured.

Typically, a clean room has a controlled level of contamination that is specified by the number of particles per cubic meter at a specified particle size (0.1 or 0.5 µm in diameter). Clean rooms are classified according to, e.g., the EN ISO 14644-1 standard (class ISO 1 to ISO 9) or the EU GMP Annex 1 standard for sterile pharmaceuticals (class A to D). In a clean room of class A/B according to EU GMP Annex 1 standard (corresponding to ISO 5) the maximally allowed number of particles (0.5 µm in diameter) amounts to about 3500, in a clean room of class C (corresponding to ISO 7) to about 3.5 x 10⁵, and in a clean room of class D (corresponding to ISO 8) to about 3.5 x 10⁶, respectively.

The maintenance of a clean environment and the prevention (or at least reduction) of further contamination is essential for the production process of the above-referenced goods. For example, in the pharmaceutical industry an aseptic environment (at least during some production steps) is inevitable for the manufacture of medicaments (cf., e.g., Schicht, H.H. (1998) Cleanrooms Int. 2, 17-28).

Clean room technology thus aims at the development of various concepts for avoiding contamination. For example, equipment inside the clean room such as furniture is designed to generate minimal air contamination. Air entering a clean room from outside is filtered to exclude dust, and the air inside is constantly re-circulated through high efficiency particulate air filters in order to remove internally generated contaminants. In addition, clean rooms may also be kept at a positive pressure so that in case of any leaks, air leaks out of the chamber instead of unfiltered air coming in.

One critical source of contamination is the transfer of materials or personnel to or from clean rooms. However, some equipment, typically made of materials having a low porosity, such as manufacturing devices (e.g. pipetting robots) or laboratory supplies (e.g., tubes, dishes) can be decontaminated (or sterilized) fairly easy by means of, e.g., autoclaving (that is, heat treatment) or gamma ray treatment.

Much more problematic is the transfer of people to, from or between different clean rooms. Personnel enter and leave clean rooms through airlocks (sometimes including an air shower stage), and wear protective clothing such as masks, gloves, shoe covers, and coveralls. However, whenever staff members move between clean rooms of different classes (e.g., for monitoring different production (and/or packaging) steps in parallel or to transport an intermediate product obtained in a given production step in another clean room where the next step takes place) have to change their complete protective clean room clothing in order to avoid microbial contaminations. This procedure is not only uncomfortable for the staff concerned but also time consuming (about 30 min per change of clothing). Since, in average, there are 3-4 coating cycles per day, the total expenditure of time can be calculated to be about 2.5 hours per day.

In addition, after each coating cycle the clean room clothing has to be disposed (in case of single use-clothing) or washed and sterilized prior to re-use. Employing single use-clothing is clearly an expensive option. Re-using the clean room clothing, on the other hand, requires considerable effort. Moreover, sterilization proceedings by heat treatment or radiation affect, at least in the medium-term, the quality and performance characteristics of the clothing material such that the re-use of such protective clothing is also limited to a clear number of cycles (cf., e.g., Gail, L. and Hortig, H.P. (2004) Reinraumtechnik, 2nd ed., Springer Verlag, Berlin, p. 319-354).

Hence, there is a continuing need for clean room clothing having improved functional properties that would not necessarily have to be changed during each and every entry in or leave of clean rooms. In order to reduce microbial contaminations clean room clothing with antimicrobial activity has previously become available. For example, the antimicrobial effect can be accomplished by incorporation of gold and/or silver fibers into the clothing material. However, the supposed advantages of this approach are still controversially discussed, particularly in view of the significantly higher costs of such equipment.

On the other hand, there is also burgeoning evidence that clean rooms are colonized by populations of specialized and highly adapted microorganisms that are capable of tolerating such extreme environmental conditions, in particular the limited nutrition supply (cf., e.g., La Duc, M.T. et al. (2007) Appl. Environ. Microbiol. 73, 2600-2611). The known and rather general inhibitory effects of, e.g., gold and silver on microbial growth may thus likely be insufficient to decontaminate clean room equipment colonized with such extremo-tolerant microorganisms.

Thus, there still remains a need for methods and compositions for controlling microbial growth on clean room equipment, particularly clean room clothing, enabling a reliable and efficient treatment even of contaminations caused by extremo-tolerant microorganism. In particular, there is a need for corresponding means allowing for the decontamination of clean room clothing in order to avoid the change of the protective clothing at each and every entry in or leave of a clean room.

Accordingly, it is an object of the present invention to provide such methods and compositions.

### SUMMARY OF THE INVENTION

In a first aspect, the present invention relates to a method for controlling microbial growth on clean room equipment, the method comprising: contacting the clean room equipment to be treated with one or more antimicrobial agents; and incubating the clean room equipment for a pre-selected period of time, wherein the one or more antimicrobial agents are of organic origin. Preferably, the clean room equipment to be treated is clean room clothing.

In specific embodiments, the step of contacting the clean room equipments comprises a physical contacting method, preferably selected from the group consisting of spraying and gassing. In alternative specific embodiments, said step of contacting comprises the chemical coupling of the one or more antimicrobial agents to the clean room equipment to be treated.

In preferred embodiments, the one or more antimicrobial agents employed are selected from the group consisting of microorganisms, particularly preferably of bacteria, yeasts, and fungi, and metabolites produced by said microorganisms. It is also preferred that the one or more of the microorganisms and/or metabolites are isolated or enriched from plants, particularly preferably from the rhizosphere, endosphere or phyllosphere of plants.

In specific embodiments, the plants from which the one or more of the microorganisms and/or metabolites are isolated or enriched are selected from the group consisting of *Fragaria spec., Brassica napus, Cucurbita pepo, Beta vulgaris, Solanum tuberosum, Sphagnum spec., Viscum album,* and *Acer tataricum.*

In a further preferred embodiment, the one or more antimicrobial agents employed comprise microorganisms of the species *Serratia plymuthica* and/or of the species *Pantoea agglomerans.*

In another preferred embodiment, the one or more antimicrobial agents comprise one or more metabolites selected from the group consisting of exoenzymes, antibiotics, volatile organic substances, and biosurfactants.

In another specific embodiment, the method according to the present invention is for controlling growth of any one or more microorganisms selected from the group consisting of *Bacillus spec., Paenibacillus spec., Geobacillus spec., Oceanobacillus spec., Micrococcus spec., Staphylococcus spec., Exiguobacterium spec., Microbacterium spec., Kocuria spec., Pseudomonas spec., Sphingomonas spec., Stenotrophomonas spec., Verticillium spec., Penicillium spec., Candida spec.,* and *Saccharomyces spec..*

In yet another specific embodiment, the method according to the present invention further comprises: removing the one or more antimicrobial agents from the clean room equipment after having performed the incubation step by means of a physical and/or chemical treatment.

In a second aspect, the present invention relates to the use of a method as defined herein for the microbial decontamination of clean room equipment, preferably of clean room clothing.

In a third aspect, the present invention relates to a kit-of-parts, comprising one or more antimicrobial agents selected from the group consisting of microorganisms, preferably bacteria, yeasts, and fungi, and metabolites produced by said microorganisms for performing the method as defined herein. Particularly preferably, the one or more antimicrobial agents employed in the kit-of-parts comprise: microorganisms of the species *Serratia plymuthica* and/or of the species *Pantoea agglomerans;* and/or one or more metabolites selected from the group consisting of exoenzymes, antibiotics, and biosurfactants.

Finally, in a fourth aspect, the present invention relates to the use of a kit-of-parts as defined herein for the microbial decontamination of clean room equipment, preferably of clean room clothing.

### DESCRIPTION OF THE DRAWINGS

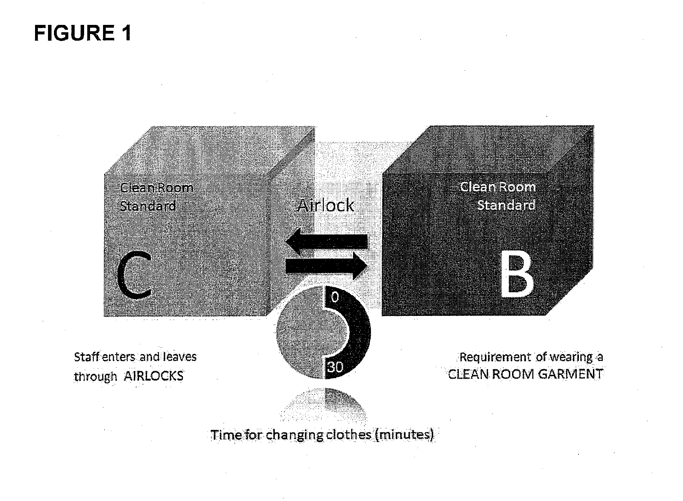

### FIGURE 1: Schematic illustration of the problem underlying the present invention.

Depicted are two clean rooms - one clean room of class C according to the EU GMP Annex 1 standard and one clean room of class B - connected by an airlock. In a clean room of class B the wearing of clean room clothing is mandatory. Staff members who would like to enter or have left clean room B have - at any single event - to change their protective clothing. The time required for such a coating cycle is about 30 minutes in average.

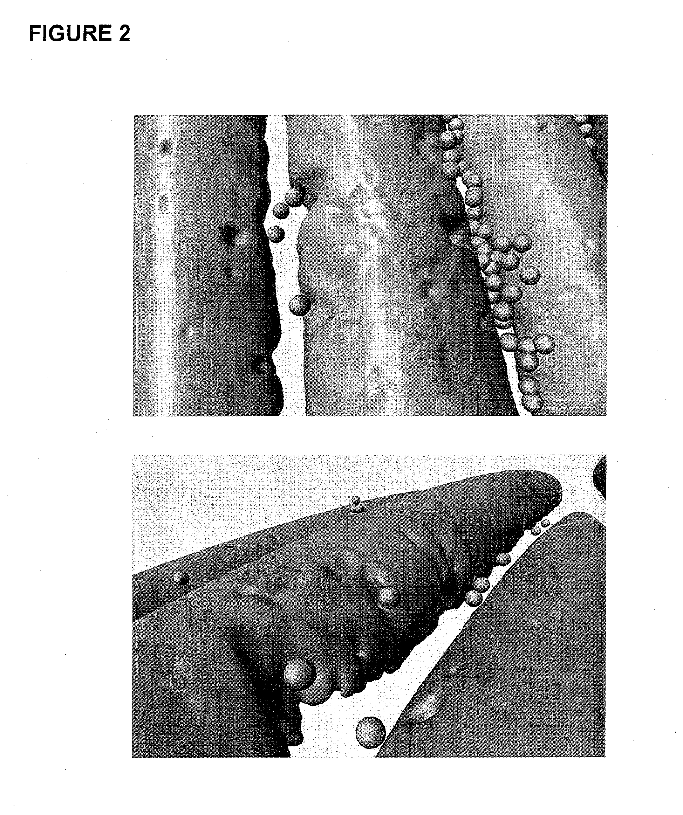

### FIGURE 2: Microbial colonization of textile surfaces.

Shown are two exemplary representations of polyester fibers colonized with *Staphylococcus epidermidis* 1457 (transformed with pASgfp_{hld}, a plasmid comprising the gfp coding sequence for further visualization). Notably, the microorganisms are mostly located at sites between the polyester fibers or in surface cavities that are difficult to access. A piece of textile (1x 1 cm) was incubated for 12 h in an overnight bacterial culture. After incubation, the textile was washed for 20 min in PBS (phosphate buffered saline) and dried for 5 min with pressure air. Subsequently, the textile was analyzed with a Confocal Laser Scanning Microscope (Leica DM 5500 Q). The resolution was 630x, and 3D volume reconstruction from the stacks was performed using Imaris software (image visualization software).

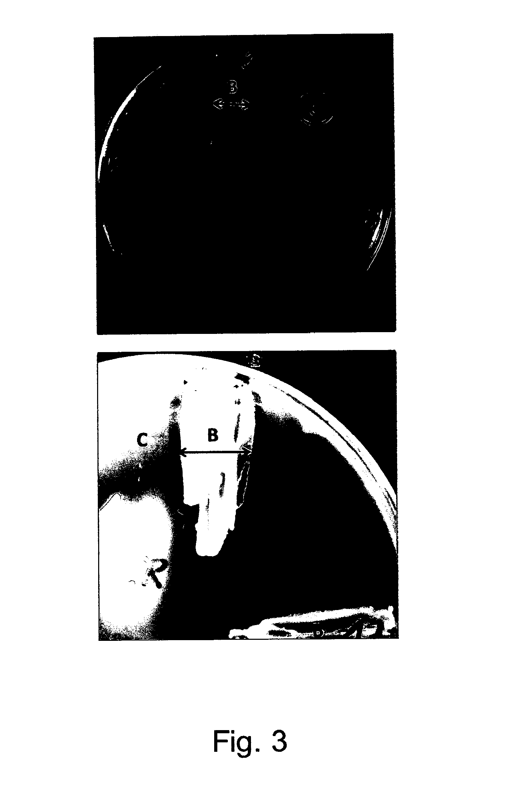

### FIGURE 3: Analysis of the efficacy of antimicrobial agents according to the invention on microbial growth.

The upper panel depicts an exemplary analysis of the efficacy of antimicrobial agents against *Verticillium dahliae.* The method (dual culture assay) was performed on Waksman agar as described in Berg, G. (1996) J. Plant Dis. Protect. 103, 20-30. The lower panel shows an exemplary *in vitro* assay of the efficacy of antimicrobial agents against *Erwinia carotovora.* The assay was modified from Rasche, F. et al (2006) J. Appl. Ecology 43, 555-566. Abbreviations: A, zone of growth inhibition; B, zone of endophytic bacteria,

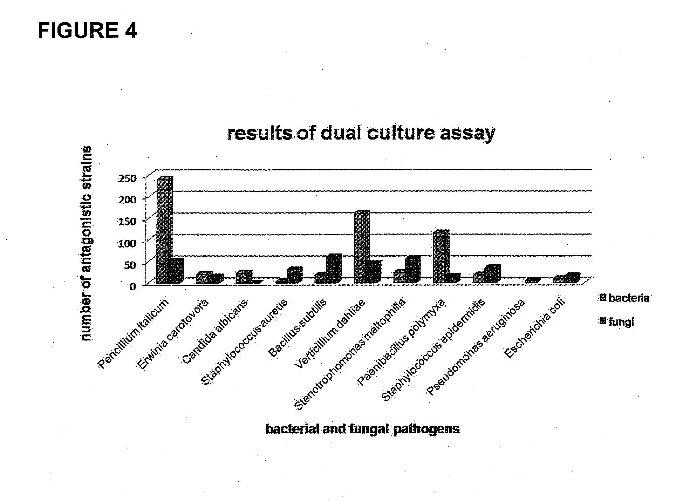

### FIGURE 4: Analysis of the efficacy of antimicrobial agents according to the invention on microbial growth.

The bar chart shows the total number of antagonists identified against eleven pathogenic fungi and bacteria known to be inhabiting clean rooms. The analysis was performed using the assay described in Fig. 3, upper panel.

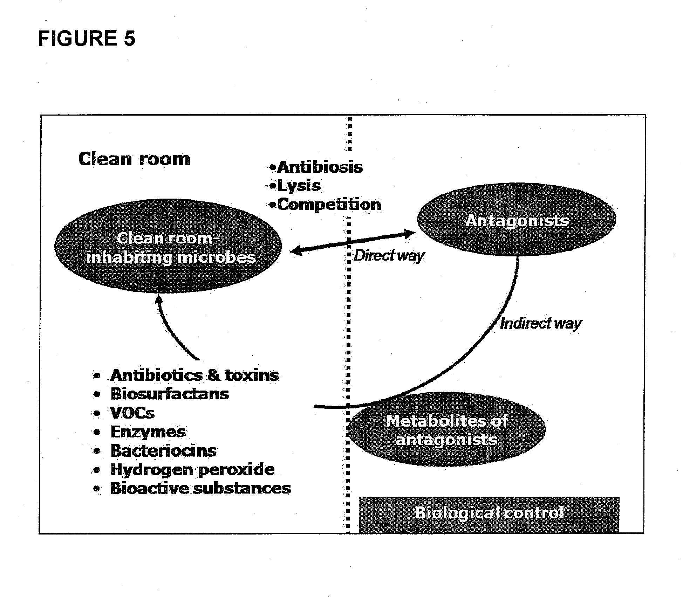

### FIGURE 5: Biological growth control of clean room-inhabiting microorganisms.

Schematic illustration of different alternatives for biologically controlling the growth of clean room-inhabiting microorganisms (microbes): (i) direct growth suppression or inhibition of the clean room-inhabiting microorganisms by one or more antagonists (i.e. antagonistic microorganisms) of the invention, e.g., via antibiosis, lysis and/or competition for nutrients; and (ii) in an indirect manner by means of metabolites of such antagonistic microorganisms such as, for example, antibiotics, toxins, biosurfactans, volatile organic compounds, (exo)enzymes, bacteriocins, biologically produced hydrogen peroxide, and other bioactive microorganisms.

### DETAILED DESCRIPTION OF THE INVENTION

The present invention is based on the unexpected finding that antimicrobial agents of organic origin which are preferably selected from microorganisms such as bacteria and fungi and from metabolites produced by said microorganisms can be successfully used for (biologically) controlling microbial growth, even of extremo-tolerant microorganisms, on clean room equipment, and particularly on clean room clothing. This, in turn, enables a reliably and efficient decontamination of clean room clothing, thus facilitating clean room operations by avoiding the otherwise necessary change of the protective clothing at each and every entry in or leave of a clean room.

The present invention will be described in the following with respect to particular embodiments and with reference to certain drawings but the invention is to be understood as not limited thereto but only by the appended claims. The drawings described are only schematic and are to be considered non-limiting.

Where the term "comprising" is used in the present description and claims, it does not exclude other elements or steps. For the purposes of the present invention, the term "consisting of' is considered to be a preferred embodiment of the term "comprising". If hereinafter a group is defined to comprise at least a certain number of embodiments, this is also to be understood to disclose a group, which preferably consists only of these embodiments.

Where an indefinite or definite article is used when referring to a singular noun e.g. "a", "an" or "the", this includes a plural of that noun unless specifically stated otherwise.

In case, numerical values are indicated in the context of the present invention the skilled person will understand that the technical effect of the feature in question is ensured within an interval of accuracy, which typically encompasses a deviation of the numerical value given of ± 10%, and preferably of ± 5%.

Furthermore, the terms first, second, third, (a), (b), (c), and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Further definitions of term will be given in the following in the context of which the terms are used. The following terms or definitions are provided solely to aid in the understanding of the invention. These definitions should not be construed to have a scope less than understood by a person of ordinary skill in the art.

In a first aspect, the present invention relates to a method for controlling microbial growth on clean room equipment, comprising:
(a) contacting the clean room equipment to be treated with one or more antimicrobial agents; and
(b) incubating the clean room equipment for a pre-selected period of time,
wherein the one or more antimicrobial agents are of organic origin.

The term "clean room equipment", as used herein, refers to any goods that are required for a production process in a clean room environment including *inter alia* furniture, any types of devices or apparatuses (e.g., pipettes, robots, centrifuges, work benches, computer systems, and the like), laboratory supplies (e.g., tubes, culture dishes, pipette tips, pens, paper, notebooks, packaging containers, and the like), and clean room clothing.

In preferred embodiments of the invention, the term "clean room equipment" refers to clean room clothing, that is, the protective clothing put on before entering a clean room. Within the present invention, the term "clean room clothing" denotes any type of garment such as, e.g., gloves, face masks, surgical caps, head covers, hairnets, full cover hoods, socks, shoe covers, undergarments, coveralls, jackets, pants, and coats. The design of such protective clothing including materials of fabrication as well as functional properties is well known in the art. In a specific embodiment, the clean room clothing used is provided with one or more antimicrobial agents of inorganic origin, for example gold and/or silver fibers incorporated in the fabrication materials.

The term "controlling microbial growth", as used herein, denotes any activity for completely inhibiting or at least reducing the growth of microorganisms such as bacteria, archaea, yeasts, fungi, viruses, and the like, in a given environment, particularly in a clean room setting. In particular, the term relates to biologically controlling microbial growth of said microorganisms, that is, by employing biological means as "growth inhibitors or growth reducing agents." The term "inhibiting", as used herein, is to be understood as not only to include the prevention of further growth of but also to killing of any given microorganisms. The term "reducing", as used herein, denotes any decrease in an microorganism's growth (or growth rate), for example, a decrease of at least 5%, 10%, 15%, 20%, 25%, 30%, 40%, 50%, 60%, 70%, 80%, 85%, 90% or 95% as compared to control conditions (i.e. in the absence of antimicrobial agents according to the present invention).

The term "antimicrobial agent", as used herein, refers to any compound or means having an inhibitory (or antagonistic) effect on the growth of microorganisms, that is, agents that are capable of at least reducing the growth rate (e.g., bacteriostatic agents with respect to controlling the growth of bacteria) as well as agents that cause toxic effects (e.g., bactericide agents killing bacteria) provided that these compounds or means are of organic origin (i.e. composed of organic compounds).

In preferred embodiments, the one or more antimicrobial agents are selected from the group consisting of microorganisms, preferably of bacteria, yeats, and fungi, and metabolites produced by said microorganisms.

"Antagonistic" microorganisms that may be employed in the present invention are characterized by one or more structural and/or functional properties conferring to them a selection advantage as compared to the microorganisms to be treated, thus resulting in a superior growth rate. Such organisms include *inter alia* (i) microorganisms that compete with the microorganisms to be treated for any nutrients and/or any trace elements (e.g., by depleting Fe³⁺ via the secretion of iron-chelating siderophores) and/or for living space, (ii) microorganisms that produce (and optionally secrete in form of exoenzymes) lytic compounds such as glucanases, chitinases, cellulases, hydrolases (e.g. lysozyme), and lyases, and (iii) microorganisms that produce antimicrobials/antibiotics, toxins, volatile organic compounds (VOCs; i.e. chemical compounds that have high enough vapor pressures under normal conditions to significantly vaporize and to enter the atmosphere) and/or biosurfactants (i.e. surface-active compounds that enhance the emulsification of hydrocarbons, have the potential to solubilize hydrocarbons and increase their availability for microbial degradation).

Examples of antibiotics include *inter alia* β-lactam antibiotics (e.g., penicillin, cephalosporin), tetracyclins, aminoglycoside (e.g., streptomycin, kanamycin, gentamicin), and macrolides (e.g., erythromycin). The term "toxins", as used herein, also includes bacteriocins, that is, proteinaceous toxins produced by bacteria to inhibit the growth of similar or closely related bacterial species. Examples of such toxins are, e.g., colicin, halocin, nisin, sakacin, and vibriocin. Examples of volatile organic compounds include *inter alia* methane and formaldehyde. Finally, examples of biosurfactants include *inter alia* mycolic acid, glycolipids, polysaccharide-lipid complexes, lipoproteins or lipopeptides, and phospholipids.

Instead of and/or in addition to the microorganisms as such one or more metabolites produced by said microorganisms, preferably the exoenzymes, antibiotics, volatile organic compounds, and biosurfactants mentioned above, may be employed for practicing the present invention. The term "produced by said microorganisms", as used herein, is not to be understood that practicing the present invention necessarily requires the isolation of any "growth inhibitory" metabolites from said microorganisms. Rather, the term merely refers to the fact that said metabolites represent "antagonistic factors" of natural origin produced (and optionally secreted) by microorganisms.

Hence, the method according to the invention may not only be performed with naturally occurring metabolites (i.e. metabolites isolated from antagonistic microorganisms as defined herein) but also with corresponding metabolites that are chemically synthesized or generated by means of recombinant gene technology (see, for example, Sambrook, J. et al. (1989) Molecular, Cloning: A Laboratory Manual, 2nd ed., Cold Spring Harbor Laboratory Press, Cold Spring Harbor, NY).

In further preferred embodiments, one or more of the microorganisms and/or metabolites are isolated or enriched from plants, particularly preferably from the rhizosphere, endosphere or phyllosphere of plants. The term "rhizosphere", as used herein, denotes the region (layer) of soil (including the root surfaces) that is directly influenced by secretions of a plant's roots and root-associated soil microorganisms. The term "endosphere", as used herein, denotes the interior of a plant. Finally, the term "phyllosphere", as used herein, refers to a plant's leaf surfaces or the total above-ground surfaces of a plant.

Examples of "antagonistic" microorganisms that can be isolated from the rhizosphere include *inter alia Chryseobacterium spec., Enterobacter spec., Salmonella typhimurium, Serratia spec., Stenotrophomonas maltophilia, Staphylococcus spec.,* and *Pseudomonas aeruginosa.* Examples of "antagonistic" microorganisms that can be isolated from the endosphere include *inter alia Staphylococcus epidermidis, Pseudomonas aeruginosa, Streptococcus mitis, Serratia spec., Enterobacter spec.,* and *Stenotrophomonas maltophilia.* Finally, examples of "antagonistic" microorganisms that can be isolated from the phyllosphere include *inter alia Serratia plymuthica, Pseudomonas putida,* and *Pantoea agglomerans.*

Such antagonistic microorganisms are further reviewed, e.g., in Hallmann, J., and Berg, G. (2006) Spectrum and population dynamics of root endophytes. In: Microbial root endophytes. Schulz, B. et al. (eds.), Springer Verlag Berlin, Heidelberg, New York, pp. 15-32.

Within the present invention, the one or more antimicrobial employed in a given setting may all be isolated or enriched from a single plant species (variety) or from at least two different plant species of the same plant genus or they may be isolated or enriched from at least two plants belonging to different genera.

In preferred embodiments, the plants are selected from the dicotyledon plants (also referred to as "dicotyledons" or "dicots"). The term "dicotyledon plants", as used herein, denotes the group of flowering plants (i.e. angiosperms) whose seed typically has two embryonic leaves or cotyledons. However, in other embodiments monocotyledon plants (also referred to as "monocotyledons" or "monocots").having only a single cotyledon may be employed as well.

In particularly preferred embodiments, the plants are selected from the group (of dicotyledons) consisting of *Fragaria spec.* (i.e. strawberry), *Brassica napus* (i.e. rape or oilseed rape), *Cucurbita pepo* (i.e. pumpkin or squash), *Beta vulgaris* (i.e. beet), *Solanum tuberosum* (i.e. potato), *Sphagnum spec.* (i.e. peat moss), *Viscum album* (i.e. mistletoe), and *Acer tataricum* (i.e. Tatar maple).

The method of the present invention may be performed with one or more antimicrobial agents isolated or enriched from any one or more plants. In preferred embodiments, the method is performed with one or more antimicrobial agents isolated or enriched from any one, any subgroup of any two or more (i.e. any two, any three, any four, any five, any six, or any seven) or all of the eight plants of the group disclosed herein above.

The term "isolated", as used herein, denotes that the antimicrobial agents used in the invention are present in pure (or almost pure) form, for example at least 75%, 80%, 85%, 90%, 95% 98% or 99% pure or completely pure (i.e. 100% pure). The term "enriched", as used herein, denotes a crude preparation (e.g., a plant extract) subjected to one or more basic purification steps (e.g., a filtration, a precipitation or a centrifugation step), thus resulting in the accumulation of the antimicrobial agents in such preparation which, however, does still include significant impurities. For example, such enriched preparations may be at least 20%, 30%, 40%, 50%, 60% or 70% pure.

The one or more antimicrobial agents employed in the present invention may comprise microorganisms of any one or more microbial species exhibiting an antagonistic (i.e. growth inhibiting or at least growth reducing) behavior towards one or more clean-room inhabiting microorganisms. Examples of such "antagonistic" microorganisms include *inter alia* any species of an organism belonging to the group consisting of the genera *Acinetobacter, Aeromonas, Agrobacterium, Amycolatopsis, Arthrobacter, Bacillus, Brevundimonas, Burkholderia, Cellulomonas, Citrobacter, Clavibacter, Chromobacterium, Comamonas, Corynebacterium, Chryseomonas, Curtobacterium, Delftia, Enterobacter, Enterococcus, Erwinia, Escherichia, Flavobacterium, Frigoribacterium, Gordonia, Herbaspirillum, Klebsiella, Kitasatospora, Kocuria, Kytococcus, Lactobacillus, Leifsonia, Lysobacter, Methylobacterium, Microbacterium, Micrococcus, Micromonospora, Morganella, Neisseria, Nocardia, Oerskovia, Paenibacillus, Pantoea, Pectobacterium, Proteus, Rahnella, Ralstonia, Rhizomonas, Salmonella, Serratia, Sphingobacterium, Sphingomonas, Staphylococcus, Streptomyces, Stenotrophomonas, Streptoverticillium, Tsukamurella, Variovorax, Xanthobacter,* and *Xanthomonas.* All these genera are well known in the art (cf., for example, Garrity, G.M. (ed.) Bergey's Manual of Systematic Bacteriology, Vol. II - The Proteobacteria (Brenner, D.J. et al., eds.), 2nd ed. (2005), Springer Press, New York).

Preferably, the method of the present invention may be performed with any species of the antagonistic microorganisms or with any combination of any one or more species belonging to any one or more genera of the antagonistic microorganisms referred to above. In other words, the method may be carried out with two or more species of microorganisms belonging to the same genus (e.g., *Bacillus, Microbacterium* or *Stenotrophomonas)* as well as with two or more species of microorganisms belonging to at least two different genera (e.g., *(Enterobacter* and *Paenibacillus)* or *(Cellulomonas* and *Neisseria* and *Xanthobacter)).*

In particularly preferred embodiments, the one or more antimicrobial agents comprise microorganisms of the species *Serratia plymuthica* and/or of the species *Pantoea agglomerans.*

In particularly preferred embodiments, the one or more antimicrobial agents comprise one or more metabolites selected from the group consisting of exoenzymes, antibiotics, volatile organic compounds, and biosurfactants.

The term "one or more" (antimicrobial agents), as used herein, denotes that the method of the invention may be performed with a single type of microbial agent (that is, e.g., a particular microorganism (i.e. a particular species) or a specific metabolite) or with at least two different types of microbial agents, wherein the at least two different types may be, e.g., at least two different species of microorganisms (that may or may not belong to the same genus) or at least two different types of metabolites (that may or may not belong to the same class of chemical compounds) or at least one microorganism and at least one metabolite. Accordingly, the method of the invention may be performed by employing, for example three different species of microorganisms (e.g., *Serratia plymuthica, Pantoea agglomerans,* and *Stenotrophomonas maltophilia),* with four different types of metabolites (e.g., a glucanase, kanamycin, and two biosurfactants) as well as with a combination of two different species of microorganisms (e.g., *Serratia plymuthica,* and *Pantoea agglomerans)* with two different types of metabolites (e.g. two biosurfactants).

The choice of (a) particular antimicrobial agent(s) for a given scenario may depend *inter alia* on the extent of microbial growth (i.e. the quantity) present on or supposed to be present on the surface the clean room equipment to be treated as well as on the nature (i.e. the quality) of the contaminants present. The skilled person is also well aware how to select one or more antimicrobial agents for a given scenario. In some embodiments, the method of the present invention thus further comprises determining and/or monitoring qualitatively and/or quantitatively the microorganisms colonizing the surfaces of the clean room equipment or be treated. Numerous methods for achieving this goal are well known in the art (cf., e.g., Gail, L. and Hortig, H.P. (2004) Reinraumtechnik, 2nd ed., Springer Verlag, Berlin, p. 344-348; Beuth Verlag GmbH (ed.); DIN EN ISO 14698-1 and 14698-2 (2004) *Cleanrooms and associated controlled environments - Biocontamination control*)*.*

Within the present invention, the term "contacting" denotes any mode of bringing spatially together the clean room equipment to be treated with the one or more antimicrobial agents.

In specific embodiments, the contacting step comprises a physical contacting method. Preferably, the physical contacting method is selected from the group consisting of spraying and gassing of the clean room equipment with the one or more antimicrobial agents (present, e.g., in gaseous form, as an aerosol, a solution or a foam), for example by means of one or more spray heads, nozzles, pump-injectors or spray valves located in an airlock adjacent a clean room).

In alternative specific embodiments, the contacting step comprises the chemical coupling of the one or more antimicrobial agents (present, e.g., in gaseous form, as an aerosol, a solution or a foam) to the clean room equipment to be treated. Chemical coupling can be achieved either by covalent or non-covalent bonding between the one or more antimicrobial agents and the respective surface(s) of the clean room equipment to be treated.

The term "covalent bonding" refers to a form of chemical bonding characterized by the sharing of one or more pairs of electrons between two components, producing a mutual attraction that holds the resultant chemical linkage (i.e. molecule) together. The term "non-covalent bonding" refers to a variety of interactions that are not covalent in nature, between molecules or parts of molecules that provide force to hold the molecules or parts of molecules together usually in a specific orientation or conformation. Such non-covalent interactions include *inter alia* ionic bonds, hydrophobic interactions, hydrogen bonds, Vander-Waals forces, and dipole-dipole bonds.

The one or more antimicrobial agents may either be attached directly to the respective surface(s) of the clean room equipment to be treated or via a linker molecule. Numerous linkers are known in the art. A skilled person is well aware of selecting a particular linker molecule appropriate for coupling a given antimicrobial agent to the equipment to be treated.

The one or more antimicrobial agents may be (chemically) coupled a functionally active form or as an inactive precursor molecule that needs to be activated, e.g., by enzymatic cleavage, photolysis, UV radiation, and the like. In some embodiments, the antimicrobial agent(s) are activated simply be releasing it from the surface(s) of the clean room equipment to which they are attached, for example via a cleavable linker molecule. In further embodiments, The one or more antimicrobial agents are incorporated or embedded in vesicles, layers, coatings or other structures on the surface of the clean room equipment from that they are released, for example, in form of a sustained release formulation over a given period of time or upon a particular trigger such as heat, radiation or the presence of microbial contaminants (i.e. the agents are incorporated in a bioactive layer). All these configurations are established in the art.

The choice of a particular contacting method may depend *inter alia* on the number, type(s) and amount(s) of the one or more antimicrobial agents to be attached, the surface properties of the clean room equipment to be treated, and the like. The skilled person is also well aware how to select an appropriate approach for a given setting.

In case of clean room clothing, the step of contacting the clothing with the antimicrobial agent(s) may be performed prior to or after having put on the clothing to be treated.

According to the present invention, after having contacted the clean room equipment to be treated with one or more antimicrobial agents said equipment is incubated in the presence of the antimicrobial agents for a pre-selected period of time.

It is apparent that the duration of the incubation step depend *inter alia* on the type(s) and concentration(s) of the one or more antimicrobial agents employed (in other words, the intensity of the treatment performed), the type(s) and number(s) of at least some of microorganisms whose growth is to be controlled (should this information be available), the size of the surface area of the clean room equipment to be treated, and the like.

The skilled person is well aware how to calculate the time period required for inhibiting the growth of or, preferably, for killing at least a substantial portion of the microbial contaminant(s). Alternatively, reference data retrieved form the scientific literature may be used. In case of clean room clothing, the duration of the incubation period will also depend on whether the method is performed prior to or after having put on the clothing to be treated. Typically, the time period is shorter if the method is performed after having put on the clothing to be treated for the sake of convenience of the personnel concerned. The overall incubation period according to the invention may last, e.g., 5 s, 10 s, 20 s, 30 s, 45 s, 1 min, 2 min, 5 min, 10 min, 20 min, 30 min, 45 min, 1 h, 1.5 h, 2 h, 3 h, 4 h, or 5 h.

In further specific embodiments, the method of the invention is combined with conventional methods for controlling microbial growth such as spraying or gassing (e.g., using hydrogen peroxide) as well as radiation (e.g., UV radiation).

In other specific embodiments, the method further comprises:

### (c) removing the one or more antimicrobial agents from the clean room equipment after the incubation period of step (b) by means of a physical and/or chemical treatment.

The term "removing", as used herein, is to be understood that, after the incubation step, the one or more antimicrobial agents are detached from the clean room equipment, for example, prior to transferring the equipment treated to a clean room. The antimicrobial agent(s) may be removed by various physical and/or chemical methods including *inter alia* heat treatment, gassing, radiation (e.g., using UV or gamma rays), enzymatic cleavage followed by a rinsing step (e.g., using distilled water), and the like. All these techniques are well established in the art.

The method according to the present invention is applicable for controlling growth of any microorganisms (i.e. contaminants) colonizing clean room equipment. Herein, such microorganisms are also referred to as "clean room-inhabiting microorganisms" or "clean room-inhabiting microbes"). The term "microorganisms", as used herein, denotes any microscopic organism (i.e. organisms too small to be seen by the naked human eye) including both prokaryotic and eukaryotic organism as well as both single cell-organisms and multi-cellular organisms. Examples of microorganisms include *inter alia* bacteria, archaea (archaebacteria), fungi, yeasts, viruses, and protists (e.g., algae, dinoflagellates, amoebae, *Plasmodium spec.,* and *Euglena spec.).*

In specific embodiments, the method according to the invention is for controlling growth of extremo-tolerant microorganisms, that is, microorganisms specifically adapted to rather harsh environmental conditions (e.g., depletion of nutrition factors, environmental stresses, and the like), preferably to the conditions occurring in clean room environments. Preferably, the microorganisms are selected from the group of bacteria, fungi, and yeasts. Numerous such extremo-tolerant microorganisms have been charcterized so far (see, for example, La Duc, M.T. et al. (2007) Appl. Environ. Microbiol. 73, 2600-2611; Moissl, C. et al (2007) FEMS Microbiol. Ecol. 61, 509-521 ).

In further specific embodiments, the method according to the invention is for controlling growth of any one or more microorganisms selected from the group consisting of *Bacillus spec., Paenibacillus spec., Geobacillus spec., Oceanobacillus spec., Micrococcus spec., Staphylococcus spec., Exiguobacterium spec., Microbacterium spec., Kocuria spec., Pseudomonas spec., Sphingomonas spec., Stenotrophomonas spec., Verticillium spec., Penicillium spec., Candida spec.,* and *Saccharomyces spec..* All these genera are well known in the art (cf., for example, Garrity, G.M. (ed.) Bergey's Manual of Systematic Bacteriology*; supra).* The method may be performed for controlling the growth of any one, any subgroup of any two or more (i.e. any two, any three, any four, any five, any six, any seven, any nine, any ten, any eleven, any twelve, any 13, any 14 or any 15) or all of the 16 microorganisms of the group disclosed herein above.

In a second aspect, the present invention relates to the use of a method as defined herein for the microbial decontamination of clean room equipment, preferably of clean room clothing. In preferred embodiments, the method is used for the complete decontamination (i.e. the inactivation and/or killing of any microorganisms colonizing the clean room equipment or inhabiting a clean room) of the clean room equipment. However, it is also possible to use the method for only interfering with the growth of one or more particular species (e.g., pathogenic organisms) that are supposed to be of critical importance for a given application.

In a second aspect, the present invention relates to a kit-of-parts, comprising one or more antimicrobial agents selected from the group consisting of microorganisms, preferably bacteria yeasts, and fungi, and metabolites produced by said microorganisms for performing the method as defined herein.

The one or more antimicrobial agents employed in the kit-of-parts according to the present invention may be any one or more of the microbial agents disclosed herein above.

In preferred embodiments, the one or more antimicrobial agents employed in the kit-of-parts comprise:
(a) microorganisms of the species *Serratia plymuthica* and/or of the species *Pantoea agglomerans;* and/or
(b) one or more metabolites selected from the group consisting of exoenzymes, antibiotics, volatile organic compounds, and biosurfactants.

The kit-of-parts according to the present invention may comprise individual containers for each of the one or more antimicrobial agents. Alternatively, two or more of the antimicrobial agents included in the kit-of-parts may be provided in a single container, optionally in a particular ratio that may depend *inter alia* on the respective functionalities, efficacies, and/or modes of action of these agents as well as on the specific application.

In specific embodiments, the one or more antimicrobial agents may be provided in form of a "ready-to-use" mixture (i.e. a particular dosage form), that is, a combination of particular agents, optionally in a particular ratio, that may be employed for a given application (e.g., the inhibition or killing of one or more specific contaminants present on clean room equipment or the treatment of a particular type of equipment).

The one or more antimicrobial agents may be provided in any form (or aggregate state) such as *inter alia* in solid or gaseous form, as an aerosol, a solution or foam. In some embodiments, the kit-of-parts comprises two or more such agents that are provided in at least two different aggregate states, e.g. as solutions and in gaseous form.

In other embodiments, at least part of the one or more antimicrobial agents is provided in a form that has to be reconstituted prior to use. For example, such agents may be provided in lyophilized form. In case of microorganisms, these may be provided as stock cultures (e.g., streaked out on agar plates, as a seed stock or "step-cultures") that have to be re-cultured prior to use. Hence, the kit-of-parts may further comprise additional reagents, e.g., buffers appropriate to dissolve lyophilized agents or culture media for cultivating microorganisms.

In some other embodiments, the kit-of-parts further comprises any devices for applying the one or more antimicrobial agents to the clean room equipment to be treated. Examples of such devices include *inter alia* spray heads, spray nozzles, pressurized dispensers, aerosol dispensers, and the like. The one or more antimicrobial agents may already be provided in these devices or may be provided separately and have to be applied to the respective devices prior to use.

Finally, in a forth aspect, the present invention relates to the use of a kit-of-as defined herein for the microbial decontamination of clean room equipment, preferably of clean room clothing.

The invention is further described by the figures and the following examples, which are solely for the purpose of illustrating specific embodiments of this invention, and are not to be construed as limiting the scope of the invention in any way.

### EXAMPLES

### Example 1: Dual culture assay

In this study, 300 endophytic bacteria and 130 endophytic fungi (isolated from different plants such as *Viscum album)* were tested for their antagonisitic potential against the following pathogens: bacteria: *Staphylococcus aureus (ATCC* 25923), *Staphylococcus epidermidis (ATCC 14990), Stenotrophomonas maltophilia (ep-15), Pseudomonas aeruginosa (QC 14-38), Escherichia coli (OP 50), Paenibacillus polymyxa (SF1-37), Bacillus subtilis (Bioindikator - EH Sus-10-7), Erwinia carotovora (Eca 549);* fungi: *Verticillium dahliae (V25), Penicillium italicum* (RB 425), and *Candida albicans (yeast).* The analysis was performed using a dual culture assay as described.

The results illustrating the antagonistic potential of the endophytic bacteria and fungi are shown in Fig. 4.

### Example 2: Bioassay to prove in vitro inhibition of bacterial and yeast pathogens:

In total, 300 endophytic bacteria and 130 endophytic fungi were tested for their antagonistic potential against the pathogens described in example 1.

For analysis of the atagonistic potential of the endophytic fungi, a dual culture assay approach was employed, which was modified from Rasche, F. et al (2006), *supra.* In brief, 5 ml of an overnight culture of one test pathogen were mixed with 15 ml of Waksman agar (containing 1.5% agar agar) and poured into a standard Petri dish. After solidification, a 4 mm² plug of each test fungus was placed on the dish. Zones of inhibition were measured after 48h.

The assay of the antagonistic potential of endophytic bacteria was performed in an analogous manner with the exception of using LB agar instead of Waksman agar. The endophytic bacteria were streaked out in broad bands. Zones of inhibition were measured after 24h.

The results illustrating the antagonistic potential of the endophytic bacteria and fungi are shown in Fig. 3, lower panel.

### Example 3: Bioassay to prove in vitro inhibition of Verticillium dahliae:

The test of the antagonistic potential of the endophytic fungi was performed on PDA using a dual culture assay modified from in Berg, G. (1996), *supra.* The plates were inoculated with 200 µl of a hyphal suspension of *Verticillium dahliae.* A 4 mm² plug of the test fungus was placed on the plates. Zones of inhibition were analyzed after 48h.

The test of the antagonistic potential of the endophytic bacteria was performed on Waksman agar (bacterial endophytes). The plates were inoculated with 200µl of a hyphal suspension of *Verticillium dahliae.* The bacteria were streaked out in broad bands. Zones of inhibition were measured after 24h.

The results illustrating the antagonistic potential of the endophytic bacteria and fungi are shown in Fig. 3, upper panel.

### Example 4: Bioassay to prove in vitro inhibition of Penicillium italicum:

The test of the antagonistic potential of the endophytic fungi was performed on PDA. Five Agar plugs with a size of 4mm² containing hyphae and spores of *Pencillium italicum* were placed on a Petri dish. Between these plugs 4 mm² sized plugs of the test fungus were placed. After 48h the growth inhibition of *Penicillium italicum* was measured.

The test of the antagonistic potential of the endophytic bacteria was performed on Waksman agar.

The present invention illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by embodiments and optional features, modifications and variations of the inventions embodied therein may be resorted to by those skilled in the art, and that such modifications and variations are considered to be within the scope of this invention.

The invention has been described broadly and generically herein. Each of the narrower species and sub-generic groupings falling within the generic disclosure also form part of the invention. This includes the generic description of the invention with a proviso or negative limitation removing any subject matter from the genus, regardless of whether or not the excised material is specifically recited herein.

Other embodiments are within the following claims. In addition, where features or aspects of the invention are described in terms of Markush groups, those skilled in the art will recognize that the invention is also thereby described in terms of any individual member or subgroup of members of the Markush group.

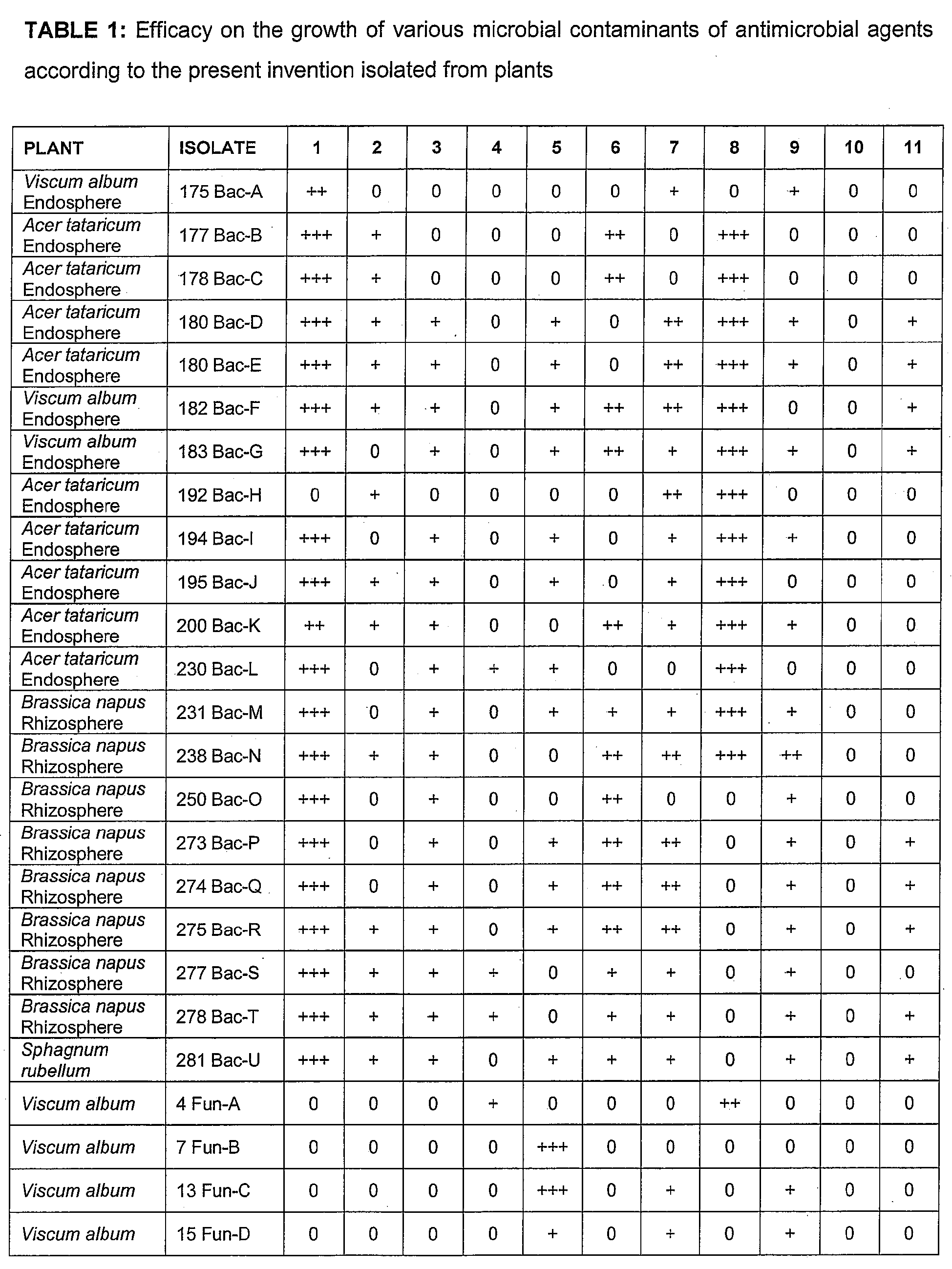

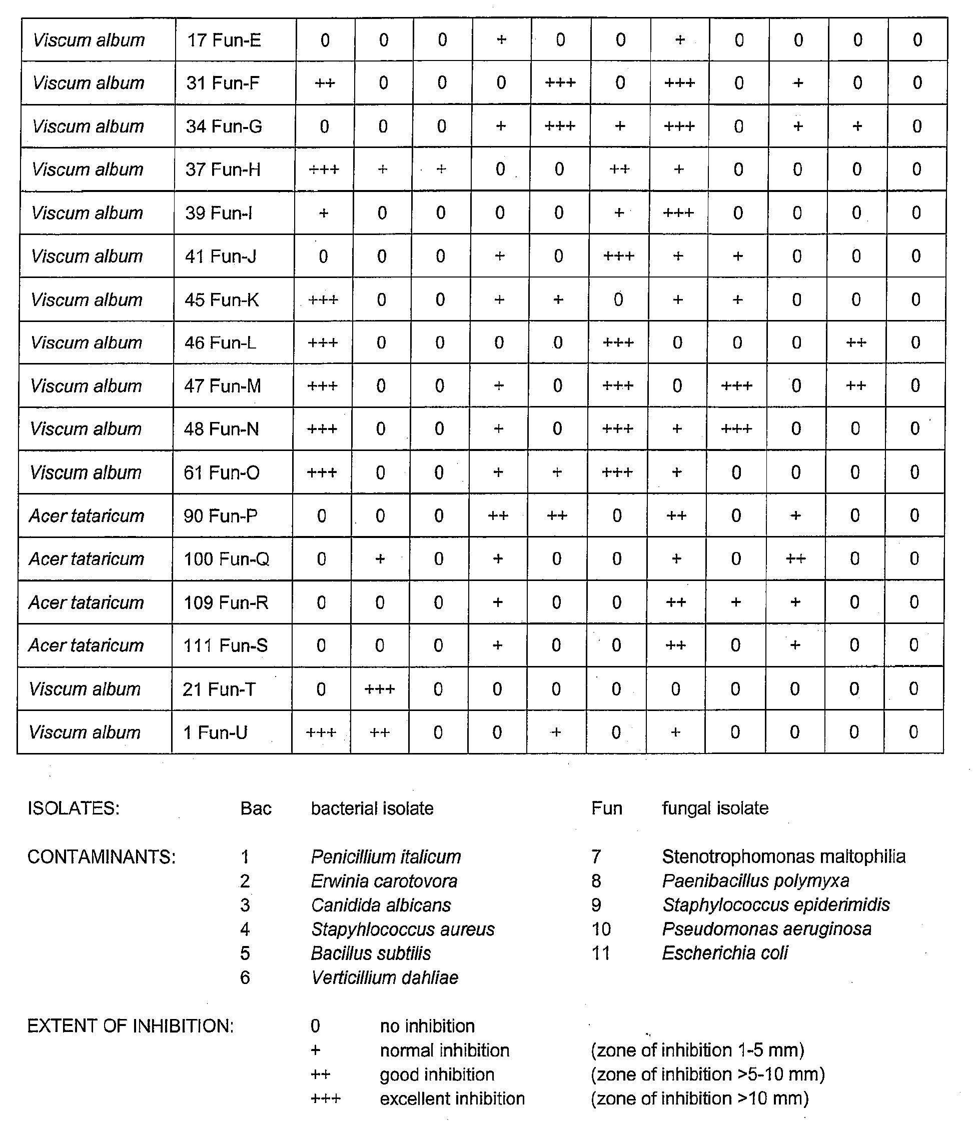

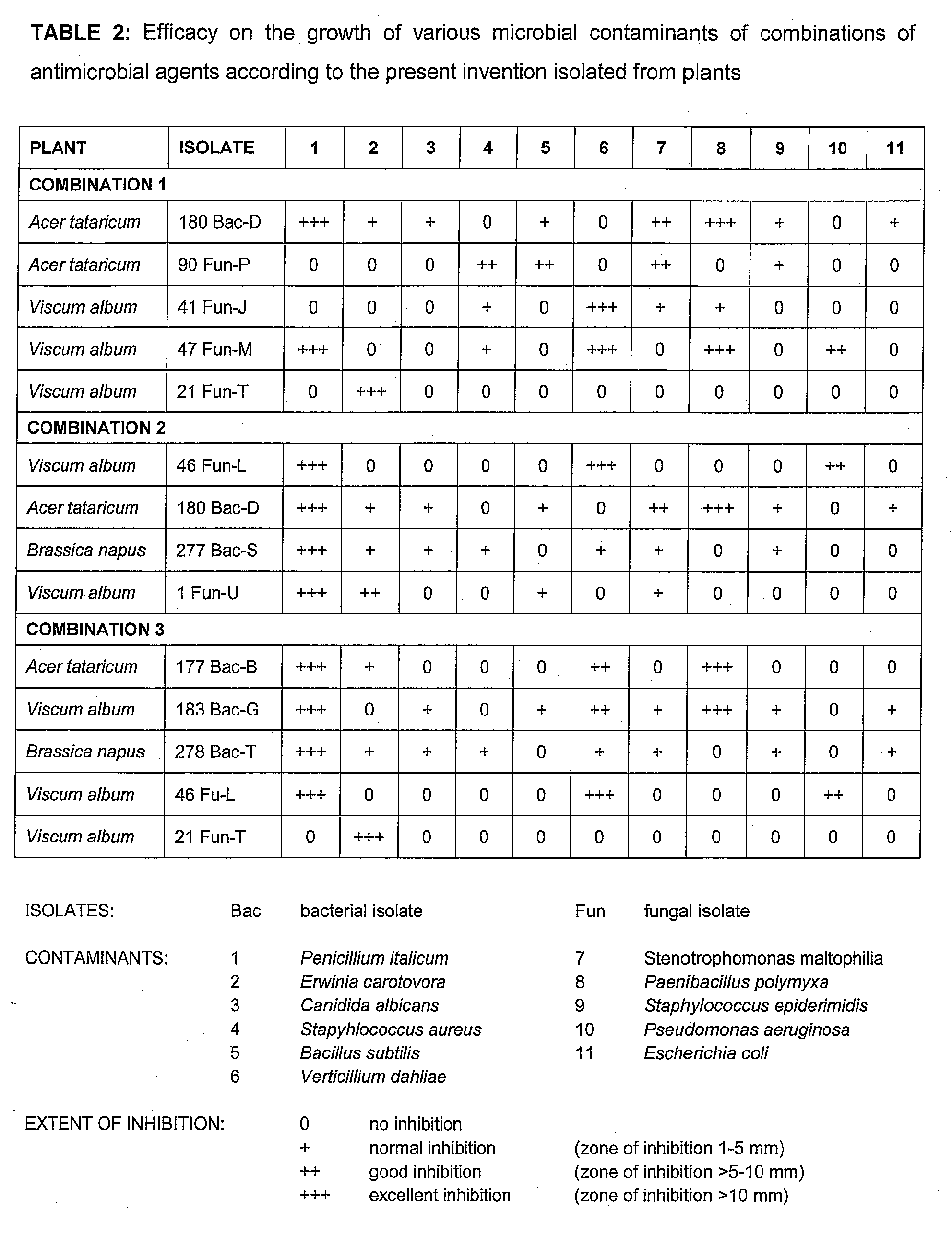

## Claims

1. Method for controlling microbial growth on clean room equipment, comprising:
(a) contacting the clean room equipment to be treated with one or more antimicrobial agents; and
(b) incubating the clean room equipment for a pre-selected period of time, wherein the one or more antimicrobial agents are of organic origin.

2. The method of claim 1, wherein the clean room equipment is clean room clothing.

3. The method of claim 1 or 2, wherein the one or more antimicrobial agents are selected from the group consisting of microorganisms, preferably of bacteria, yeasts and fungi, and metabolites produced by said microorganisms.

4. The method of claim 3, wherein one or more of the microorganisms and/or metabolites are isolated or enriched from plants, preferably from the rhizosphere, endosphere or phyllosphere of plants.

5. The method of claim 4, wherein the plants are selected from the group consisting of *Fragaria spec., Brassica napus, Cucurbita pepo, Beta vulgaris, Solanum tuberosum, Sphagnum spec., Viscum album,* and *Acer tataricum.*

6. The method of any of claims 1 to 5, wherein the one or more antimicrobial agents comprise microorganisms of the species *Serratia plymuthica* and/or of the species *Pantoea agglomerans.*

7. The method of any of claims 1 to 6, wherein the one or more antimicrobial agents comprise one or more metabolites selected from the group consisting of exoenzymes, antibiotics, volatile organic substances, and biosurfactants.

8. The method of any of claims 1 to 7, for controlling growth of any one or more microorganisms selected from the group consisting of *Bacillus spec., Paenibacillus spec., Geobacillus spec., Oceanobacillus spec., Micrococcus spec., Staphylococcus spec., Exiguobacterium spec., Microbacterium spec., Kocuria spec., Pseudomonas spec., Sphingomonas spec., Stenotrophomonas spec., Verticillium spec., Penicillium spec., Candida spec.,* and *Saccharomyces spec..*

9. The method of any of claims 1 to 8, further comprising:
(c) removing the one or more antimicrobial agents from the clean room equipment after the incubation period of step (b) by means of a physical and/or chemical treatment.

10. The method of any of claims 1 to 9, wherein step (a) comprises a physical contacting method, preferably selected from the group consisting of spraying and gassing.

11. The method of any of claims 1 to 9, wherein step (a) comprises the chemical coupling of the one or more antimicrobial agents to the clean room equipment to be treated.

12. Use of a method as defined in any of claims 1 to 11 for the microbial decontamination of clean room equipment, preferably of clean room clothing.

13. Kit-of-parts, comprising one or more antimicrobial agents selected from the group consisting of microorganisms, preferably bacteria, yeasts, and fungi, and metabolites produced by said microorganisms for performing the method as defined in any of claims 1 to 11.

14. The kit-of-parts of claim 13, wherein the one or more antimicrobial agents comprise:
(a) microorganisms of the species *Serratia plymuthica* and/or of the species *Pantoea agglomerans;* and/or
(b) one or more metabolites selected from the group consisting of exoenzymes, antibiotics, volatile organic substances, and biosurfactants.

15. Use of a kit-of-parts of claim 13 or 14 for the microbial decontamination of clean room equipment, preferably of clean room clothing.
